# EUROPÄISCHE PATENTSCHRIFT

(11) **EP 1 135 075 B1**
(45) Veröffentlichungstag und Bekanntmachung des Hinweises auf die Patenterteilung: **01.09.2004**
(21) Anmeldenummer: 99958101.0
(22) Anmeldetag: 20.11.1999
(51) Int. Cl.: A61B 17/68

(54) **DISTRAKTIONSVORRICHTUNG**
DISTRACTION ASSEMBLY
DISPOSITIF DE DISTRACTION

(30) Priorität: 04.12.1998 DE 19856062
(43) Veröffentlichungstag der Anmeldung: 26.09.2001
(73) Patentinhaber: Wittenstein AG, 97999 Igersheim (DE)
(72) Erfinder: STAUCH, Roman, D-97959 Assamstadt (DE); KLEIN, Jürgen, D-97990 Weikersheim (DE)
(74) Vertreter: Weiss, Peter, Dr.
(86) Internationale Anmeldenummer: PCT/EP1999/008969
(87) Internationale Veröffentlichungsnummer: WO 2000/033751

(56) Entgegenhaltungen:
- WO-A-98/09577
- WO-A-98/47438
- WO-A-99/51160
- DE-A- 19 741 757
- FR-A- 2 267 080
- US-A- 5 626 579
- US-A- 5 672 177

## Beschreibung

Die Erfindung betrifft eine Distraktionsvorrichtung zur plastischen Gesichtschirurgie, zur Korrektur eines Oberund/oder Unterkiefers an einem Schädel bspw. zur Beseitigung eines Überbisses, Kreuzbisses, eines Gaumenspaltes oder zur Überbrückung von bspw. durch Krankheit entnommenen Knochenstruktur mit einer Einrichtung aus wenigstens zwei axial gegeneinander bewegbaren Elementen.

Korrekturen am Kiefer, am Oberkiefer und/oder Unterkiefer, werden heute meist durch eine Operation durchgeführt. Diese Operation dauert ca. 2 bis 3 Stunden und kann nur in stationären Behandlungen durchgeführt werden. Eine Hospitalisierung ist daher unvermeidlich notwendig.

Abgesehen von der erforderlichen, aufwendigen Operation stellt sich oft der Nachteil heraus, dass häufige Korrekturen nur immer durch weitere folgende operative Eingriffe möglich sind. Dies ist für den Patient eine zusätzliche Belastung, da derartige Eingriffe nicht ohne erheblichen Aufwand betrieben werden können.

Häufige Erkrankungen des Kiefers, insbesondere auch des Unterkiefers, verursacht durch Tumore, auch in den Bereichen der Zähne und Zahnwurzeln, insbesondere auch durch Fisteln können eine Schädigung der Knochenstruktur des Kiefers verursachen.

Erforderlich sind dann Eingriffe, die auch teilweise zum Verlust der Knochenstruktur führen. Dieser Verlust muss bspw. durch entsprechende Vorrichtungen und das langwierige Wachstum ausgeglichen werden. Hierzu ist es erforderlich Korrekturen nach einem Zusammenwachsen vorzunehmen, um eine Feinabstimmung bspw. des Unterkiefers gegen den Oberkiefer vorzunehmen. Unerwünscht sind derartige operative Nachbehandlungen, da der Patient hierdurch sehr belastet wird. Auch gesichtschirurgische Operationen sind nach Abschluss derartigen Behandlungen häufig die Folge, was unerwünscht ist.

Durch ein häufiges Nachoperieren können zudem Infektionsherde entstehen.

Eine Distraktionsvorrichtung gemäß Oberbegriff des Anspruchs 1 ist z.B. aus US-A-5672 177 bekannt geworden.

Der vorliegenden Erfindung liegt die Aufgabe zugrunde eine Distraktionsvorrichtung zu schaffen, welchen die genannten Nachteile beseitigt und mit welcher, insbesondere ein stufenloses, zeitunabhängiges Auseinanderbewegen zweier Kieferteile möglich ist. Dabei sollen auf sehr einfache Weise grössere Korrekturbereiche bei geringerer humaner Belastung und insbesondere auch Systembelastung möglich sein.

Zur Lösung dieser Aufgabe führt, dass die Einrichtung berührungslos mittelbar oder unmittelbar betätigbar ist.

Bei der vorliegenden Erfindung wird insbesondere eine Distraktionsvorrichtung subkutan mittelbar oder unmittelbar betrieben, berührungslos von aussen betätigt und in einen Bereich eines Kiefers eingesetzt. Dabei weist die Distraktionsvorrichtung eine Einrichtung auf, welche vorzugsweise aus zwei gegeneinander axial bewegbaren Elementen, vorzugweise Zylindern gebildet ist. Endseits sind Aufnahmen vorgesehen, durch welche Befestigungselemente eingreifen können. Diese durchgreifen die Einrichtung und legen die Distraktionsvorrichtung am Kiefer fest. Die Befestigungselemente sind wiederlösbar mit dem Kiefer verbunden, so dass nach einer erfolgten Distraktionsbehandlung die Einrichtung operativ wieder entfernt werden kann.

Im Rahmen der Erfindung soll auch liegen, dass einzelne Segmente des Knochens bzw. des Kiefers an der Distraktionsvorrichtung festgelegt und verschoben werden können. Hierzu können an beliebigen Stellen am Element Aufnahmen für Befestigungselemente vorgesehen sein. Ein Knochensegment kann durch bspw. Fehlstellen aufgrund von Tumorentfernung gehalten und/oder verschoben werden.

Von Bedeutung ist jedoch bei der vorliegenden Erfindung, dass eine permanente Distraktion und ein Auseinanderbewegen zweier Kieferteile, die über einen Spalt getrennt sind berührungslos von aussen erfolgen kann. Dies geschieht mittels Induktion, in dem ein Energieübertragungselement die Einrichtung vorzugsweise induktiv von aussen in Betrieb setzt. Hierzu kann eine Betätigungseinrichtung intern oder extern der Einrichtung der Distraktionsvorrichtung zugeordnet sein.

Ebenso liegt im Rahmen der vorliegenden Erfindung, dass auch das Energieübertragungselement entweder extern an die Betätigungseinrichtung anschliesst oder intern in der Einrichtung der Distraktionsvorrichtung vorgesehen ist. Bei der externen Ausführung kann die Einrichtung und deren axial gegeneinander bewegbaren Elemente grösser ausgebildet sein, da von extern die Betätigungseinrichtung und die Übertragungseinrichtung diese in Betrieb setzen. Diese stehen über eine Verbindungsleitung miteinander in Verbindung. Wichtig ist jedoch, dass die Betätigungseinrichtung sowie das Energieübertragungselement ebenfalls subkutan implantiert sind. Gerade im Kieferbereich, in welchem nur sehr wenig Raum für das Implantieren von Betätigungseinrichtung und Energie-übertragungseinrichtung vorgesehen sind, hat es sich als besonders günstig erwiesen, diese an einem anderen Ort im Körper zu implantieren. Bspw. ist der Brust- oder Bauchbereich dafür sehr geeignet. Lediglich eine sehr dünne Verbindungsleitung stellt zur Betätigung der Einrichtung die notwendige Verbindung her. Induktiv kann dann über das Energieübertragungselement die Distraktionsvorrichtung in Betrieb genommen werden. Dies kann in einzelnen Behandlungen erfolgen, wobei eine derartige Korrektur permanent immer vorgenommen werden kann ohne dass eine operative Behandlung des Patienten notwendig ist.

Ferner ist daran gedacht, das Energieübertragungselement auch in der Nähe oder auch in ganz anderen Körperbereichen unter der Haut unterzubringen, um die Einrichtung in Betrieb zu nehmen. Bevorzugt wird die Einrichtung über Druck hydraulisch angetrieben, wobei entsprechende Antriebsmittel, Kolben od. dgl. in der Betätigungseinrichtung bspw. elektromechanisch bewegt werden. Hydraulisch können dann die Elemente der Einrichtung auseinander bewegt werden. Die Einrichtung kann auch mittels piezobetriebenen Aktuatoren oder Aktuatoren aus Formgedächtnislegierungen betrieben werden.

Es hat sich zudem als besonders günstig erwiesen, die Korrekturen bzw. die Distraktion in mehreren aufeinanderfolgenden Zeitabständen kontinuierlich vorzunehmen, da die Geweberelaxion bzw. der langsame biologische Adaptionsprozess dies zur schnelleren Genesung erfordert.

Ferner ist von Vorteil, dass bei der vollständigen subkutanen implantierten Distraktionsvorrichtung eine Distraktion, bspw. unter Röntgen kontrolliert und nachgestellt werden kann. Die Korrekturen können auf diese Weise überprüft werden, so dass der Kiefer seine gewünschte Form erhält.

Die vorliegende Erfindung bietet langfristige Möglichkeiten, Distraktionsvorrichtungen ohne zusätzliche operative Eingriffe zu betreiben. Dies ist insbesondere dann von Vorteil, wenn häufig Korrekturen auch bei einer sehr langsamen Knochenneubildung oder bedingt durch das Wachstum, bspw. von Kindern, erforderlich sind.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine schematisch dargestellte Draufsicht auf eine erfindungsgemässe Distraktionsvorrichtung in einer Gebrauchslage, eingesetzt in einen Unterkiefer;
Figur 2 eine schematisch dargestellte Draufsicht auf die Distraktionsvorrichtung gemäss Figur 1 in Gebrauchslage mit separater Betätigungseinrichtung.

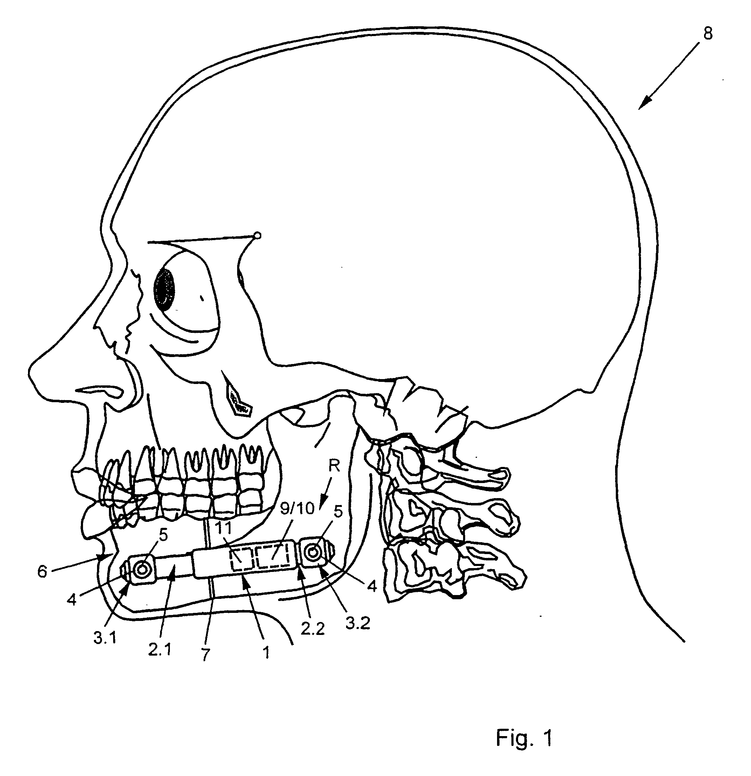

Gemäss Figur 1 weist eine erfindungsgemässe Distraktionsvorrichtung R eine Einrichtung 1 auf, welche aus vorzugsweise zwei gegeneinander axial bewegbaren Elementen 2.1, 2.2 besteht. Im bevorzugten Ausführungsbeispiel sind die Elemente 2.1, 2.2 zylinderartig und ineinander verschiebbar ausgebildet. Hier soll jedoch der Erfindung keine Grenze gesetzt sein. Bspw. können auch gegeneinander verschiebbare Rechteckprofile, Schienenprofile od. dgl. vorgesehen sein.

Endseits weisen die Elemente 2.1, 2.2 Aufnahmen 3.1, 3.2 auf, die mit einer hier nicht näher dargestellten Öffnung 4 versehen sind, durch welche ein Befestigungselement 5 greift. Das Befestigungselement 5 lässt sich vorzugsweise quer zur Einrichtung 1 in einen Kiefer 6, insbesondere Unterkiefer wiederlösbar einsetzen. Es soll auch daran gedacht sein eine Mehrzahl von Aufnahmen 3.1, 3.2 an dem Element 2.1 und/oder Element 2.2 an beliebigen Stellen, ggf. verschiebbar zum Festlegen und/oder Verschieben von Knochensegmenten vorzusehen.

Dabei wird die Einrichtung 1 subkutan in den Kiefer 6 eines Schädels 8 operativ eingesetzt. Im vorliegenden Ausführungsbeispiel enthält die Einrichtung 1 eine Betätigungseinrichtung 9 und ggf. ein Energieübertragungselement 10. Die Betätigungseinrichtung 9 wird von dem Energie-übertragungselement 10 mit Energie versorgt und gesteuert. Sie betätigt ein Antriebsmittel 11, welches die beiden Elemente 2.1, 2.2 der Einrichtung 1 auseinanderbewegt.

Im vorliegenden Ausführungsbeispiel sind Betätigungseinrichtung 9, Energieübertragungselement 10 und Antriebsmittel 11 nur schematisch dargestellt. Das Energieübertragungselement 10 kann aus einem Sendekopf, dem sogenannten Transmitter und dem subkutan implantierten Empfänger dem sogenannten Receiver bestehen. Der Sendekopf ist im vorliegenden Ausführungsbeispiel nicht aufgezeigt und kann an den Receiver herangeführt werden, um die Einrichtung 1 in Betrieb zu setzen. Auf diese Weise lässt sich die Einrichtung 1, insbesondere deren Elemente 2.1, 2.2 kontinuierlich und insbesondere berührungslos auseinanderbewegen, so dass ein Spalt 7 erweiterbar ist. Das Auseinanderbewegen lässt sich stufenweise und insbesondere stufenlos vornehmen, ohne dass ein operativer Eingriff zur weiteren Distraktion erforderlich ist.

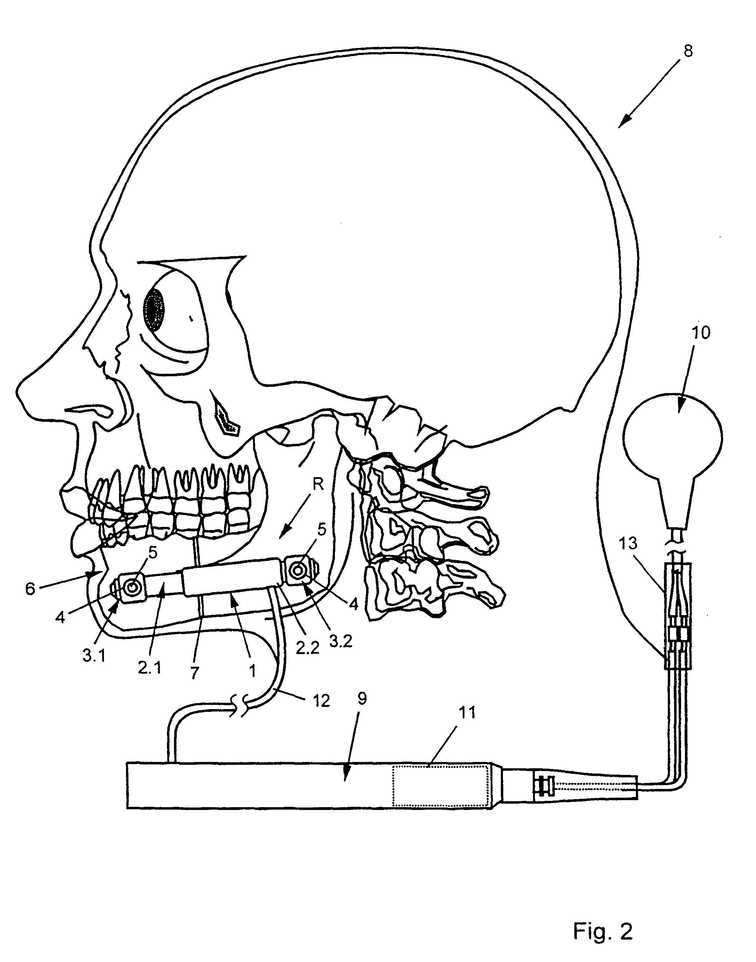

Werden sehr hohe Distraktionskräfte gefordert und ist die Distraktionsvorrichtung R grösser dimensioniert, so kann die Betätigungseinrichtung 9, wie insbesondere in Figur 2 dargestellt, auch ausserhalb der Einrichtung 1 angeordnet sein. Dabei stellt eine Verbindungsleitung 12, die von beliebiger Länge sein kann, eine Verbindung zwischen der Einrichtung 1 und der Betätigungseinrichtung 9 her. Die Einrichtung 1 ist wie oben beschrieben aus zwei vorzugsweise axial gegeneinander bewegbaren Elementen 2.1, 2.2 gebildet.

Wichtig bei der vorliegenden Erfindung ist jedoch, dass Betätigungseinrichtung 9 und Verbindungsleitung 12, ebenfalls subkutan implantierbar sind, wobei diese bspw. in einem anderen Körperbereich, im Brust-, Bauch-, oder sogar Rückenbereich angeordnet sind. Bevorzugt schliesst an die Betätigungseinrichtung 9 das Energieübertragungselement 10 an, welches ebenfalls subkutan im Körper implantiert ist. Von aussen kann dann berührungslos und induktiv über das Energieübertragungselement 10 der Betätigungseinrichtung 9 zum Steuern und Betreiben Energie zugeführt werden.
Das hier gestrichelt dargestellte Antriebsmittel 11 liefert in der Betätigungseinrichtung 9 den erforderlichen Druck, um die beiden Elemente 2.1, 2.2, insbesondere Zylinder auseinander zu bewegen. Eine Distraktion im Spalt 7 erfolgt.

Eine weitere Besonderheit stellt eine Verbindungsstelle 13 zwischen Energieübertragungselement 10 und Betätigungseinrichtung 9 dar. Diese Verbindungsstelle 13 lässt ein schnelles Auswechseln des Energieübertragungselementes 10 zu. Dieses kann bspw. bei den unterschiedlichsten Distraktionsvorrichtungen R in beliebiger Länge an die Betätigungseinrichtung 9 angeschlossen werden. Hierdurch lässt sich insgesamt die Distraktionsvorrichtung R universeller einsetzen, so dass auch das Energieübertragungselement 10 an sehr günstige Körperbereiche zur Energieübertragung und Steuerung eingesetzt werden kann.

| Positionszahlenliste | | | | | |
|---|---|---|---|---|---|
| 1 | Einrichtung | 34 | | 67 | |
| 2 | Element | 35 | | 68 | |
| 3 | Aufnahme | 36 | | 69 | |
| 4 | Öffnung | 37 | | 70 | |
| 5 | Befestigungselement | 38 | | 71 | |
| 6 | Kiefer | 39 | | 72 | |
| 7 | Spalt | 40 | | 73 | |
| 8 | Schädel | 41 | | 74 | |
| 9 | Betätigungseinrichtung | 42 | | 75 | |
| 10 | Energieübertragungselement | 43 | | 76 | |
| 11 | Antriebsmittel | 44 | | 77 | |
| 12 | Verbindungsleitung | 45 | | 78 | |
| 13 | Verbindungsstelle | 46 | | 79 | |
| 14 | | 47 | | | |
| 15 | | 48 | | | |
| 16 | | 49 | | R | Distraktionsvorrichtung |
| 17 | | 50 | | | |
| 18 | | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Distraktionsvorrichtung zur plastischen Gesichtschirurgie, zur Korrektur eines Ober- und/oder Unterkiefers an einem Schädel (8) bspw. zur Beseitigung eines Überbisses, Kreuzbisses, eines Gaumenspaltes oder zur Überbrückung von bspw. durch Krankheit entnommenen Knochenstruktur mit einer Einrichtung (1) aus wenigstens zwei axial gegeneinander bewegbaren Elementen (2.1, 2.2), die subkutan vollimplatierbar und einsetzbar ist,
**dadurch gekennzeichnet,**
**dass** die Einrichtung (1) berührungslos mittelbar oder unmittelbar betätigbar ist.

2. Distraktionsvorrichtung nach Anspruch 1, **dadurch gekennzeichnet, dass** an die Einrichtung (1) eine Betätigungseinrichtung (9) anschliesst, welche ausserhalb eines Kieferbereiches subkutan im Körper implantierbar ist.

3. Distraktionsvorrichtung nach Anspruch 2, **dadurch gekennzeichnet, dass** die Betätigungseinrichtung (9) über eine Verbindungsleitung (12) mit der Einrichtung (1) in Verbindung steht.

4. Distraktionsvorrichtung nach Anspruch 2 oder 3, **dadurch gekennzeichnet, dass** die Betätigungseinrichtung (9) und/oder die Einrichtung (1) mit einem Energieübertragungselement (10) versehen ist, an welches berührungslos Energie von aussen, ggf. induktiv zuführbar ist.

5. Distraktionsvorrichtung nach wenigstens einem der Ansprüche 2 bis 4, **dadurch gekennzeichnet, dass** die Betätigungseinrichtung (9) externer oder interner Bestandteil der Einrichtung (1) ist.

6. Distraktionsvorrichtung nach Ansprpuch 4 oder 5, **dadurch gekennzeichnet, dass** das Energieübertragungselement (10) externer oder interner Bestandteil der Einrichtung (1) oder der Betätigungseinrichtung (9) ist.

7. Distraktionsvorrichtung nach wenigstens einem der Ansprüche 1 bis 6, **dadurch gekennzeichnet, dass** die Elemente (2.1, 2.2) der Einrichtung (1) mechanisch, elektromechanisch, pneumatisch oder hydraulisch gegeneinander bewegbar sind.

8. Distraktionsvorrichtung nach wengistens einem der Ansprüche 1 bis 7, **dadurch gekennzeichnet, dass** die Elemente (2.1, 2.2) zylinderartig ausgebildet und ineinander verfahrbar sind.

9. Distraktionsvorrichtung nach wenigstens einem der Ansprüche 1 bis 8 **dadurch gekennzeichnet, dass** die Elemente (2.1, 2.2) endseits mit Aufnahmen (3.1, 3.2) versehen sind, in welche jeweils Befestigungselemente (5) zum wiederlösbaren Festlegen der Einrichtung (1) an einem Kiefer (6) eingreifen.

10. Distraktionsvorrichtung nach Anspruch 9, **dadurch gekennzeichnet, dass** das Auseinanderbewegen der Elemente (2.1, 2.2) kontinuierlich, stufenlos erfolgen kann.

11. Distraktionsvorrichtung nach wenigstens einem der Ansprüche 2 bis 10, **dadurch gekennzeichnet, dass** die Einrichtung (1) oder die Betätigungseinrichtung (9) zumindest ein Antriebsmittel (11) zur Erzeugung des pneumatischen Druckes zum Auseinanderbewegen der Elemente (2.1, 2.2) aufweist.

12. Distraktionsvorrichtung nach wenigstens einem der Ansprüche 1 bis 11, **dadurch gekennzeichnet, dass** über Piezoaktoren oder Aktoren aus Formgedächtnislegierungen die Elemente (2.1, 2.2) gegeneinander bewegbar sind.

13. Distraktionsvorrichtung nach wenigstens einem der Ansprüche 9 bis 12, **dadurch gekennzeichnet**, eine Mehrzahl von Aufnahmen 3.1, 3.2 an dem Element 2.1 und/oder Element 2.2 an beliebigen Stellen, ggf. verschiebbar zum Festlegen und/oder Verschieben von Knochensegmenten vorgesehen sind.

## Claims

1. Distraction device for facial plastic surgery, for correcting an overshot or undershot jaw on a skull (8), for example to correct a protruding bite, a crosswise bite or a cleft palate, or for bridging-over a bone structure which has, for example, been removed because of illness, said device having a means (1) formed from at least two elements (2.1, 2.2), which are axially displaceable towards each other, and which means is fully implantable and insertable subcutaneously, **characterised in that** the means (1) is indirectly or directly actuatable in a contactless manner.

2. Distraction device according to claim 1, **characterised in that** an actuating means (9) communicates with the means (1) and is implantable subcutaneously in the body outside a jaw region.

3. Distraction device according to claim 2, **characterised in that** the actuating means (9) communicates with the means (1) via a connecting line (12).

4. Distraction device according to claim 2 or 3, **characterised in that** the actuating means (9) and/or the means (1) are/is provided with an energy transmitting element (10), to which energy is suppliable from externally, possibly inductively, in a contactless manner.

5. Distraction device according to least one of claims 2 to 4, **characterised in that** the actuating means (9) is an external or an internal component part of the means (1).

6. Distraction device according to claim 4 or 5, **characterised in that** the energy transmitting element (10) is an external or an internal component part of the means (1) or of the actuating means (9).

7. Distraction device according to at least one of claims 1 to 6, **characterised in that** the elements (2.1, 2.2) of the means (1) are displaceable towards each other mechanically, electromechanically, pneumatically or hydraulically.

8. Distraction device according to at least one of claims 1 to 7, **characterised in that** the elements (2.1, 2.2) are cylindrical and displaceable one inside the other.

9. Distraction device according to at least one of claims 1 to 8, **characterised in that** the elements (2.1, 2.2) are terminally provided with receiving means (3.1, 3.2), in which securing elements (5) respectively engage for the re-releasable securement of the means (1) on a jaw (6).

10. Distraction device according to claim 9, **characterised in that** the elements (2.1, 2.2) can be moved apart continuously and steplessly.

11. Distraction device according to at least one of claims 2 to 10, **characterised in that** the means (1) or the actuating means (9) has at least one driving means (11) for generating the pneumatic pressure for moving the elements (2.1, 2.2) apart.

12. Distraction device according to at least one of claims 1 to 11, **characterised in that** the elements (2.1, 2.2) are displaceable towards each other via piezo-electric actuators or actuators formed from form-memorising alloys.

13. Distraction device according to at least one of claims 9 to 12, **characterised in that** a plurality of receiving means (3.1, 3.2) are provided on the element (2.1) and/or the element (2.2) at any desirable locations, possibly in a displaceable manner for the securement and/or displacement of the bone segments.

## Revendications

1. Dispositif de distraction pour la chirurgie plastique du visage, pour la correction d'une mâchoire supérieure et/ou inférieure d'un crâne (8), par exemple, pour éliminer une surocclusion, une occlusion croisée, une fente du palais ou pour le pontage d'une structure osseuse enlevée, par exemple, par suite d'une maladie, à l'aide d'un dispositif (1) composé au moins de deux éléments (2-1, 2.2) déplaçables axialement l'un par rapport à l'autre et qui peut être totalement implanté et utilisé de manière sous-cutanée,
**caractérisé par le fait**
**que** le dispositif (1) peut être actionné indirectement ou directement sans contact.

2. Dispositif de distraction selon la revendication 1, **caractérisé par le fait qu'**au dispositif (1) est raccordé un dispositif d'actionnement (9) pouvant être implanté dans le corps, de manière sous-cutanée à l'extérieur d'une zone de mâchoire.

3. Dispositif de distraction selon la revendication 2, **caractérisé par le fait que** le dispositif d'actionnement (9) communique par l'intermédiaire d'une ligne de connexion (12) avec le dispositif (1).

4. Dispositif de distraction selon la revendication 2 ou 3, **caractérisé par le fait que** le dispositif d'actionnement (9) et/ou le dispositif (1) est muni d'un élément de transmission d'énergie (10) vers lequel peut être alimentée sans contact de l'énergie de l'extérieur, éventuellement de manière inductive.

5. Dispositif de distraction selon au moins l'une des revendications 2 à 4, **caractérisé par le fait que** le dispositif d'actionnement (9) est un composant externe ou interne du dispositif (1).

6. Dispositif de distraction selon la revendication 4 ou 5, **caractérisé par le fait que** l'élément de transmission d'énergie (10) est un composant externe ou interne du dispositif (1) ou du dispositif d'actionnement (9).

7. Dispositif de distraction selon au moins l'une des revendications 1 à 6, **caractérisé par le fait que** les éléments (2.1, 2.2) du dispositif (1) peuvent être déplacés de manière mécanique, électromécanique, pneumatique ou hydraulique l'un par rapport à l'autre.

8. Dispositif de distraction selon au moins l'une des revendications 1 à 7, **caractérisé par le fait que** les éléments (2.1, 2.2) sont réalisés de forme cylindrique et déplaçables l'un dans l'autre.

9. Dispositif de distraction selon au moins l'une des revendications 1 à 8, **caractérisé par le fait que** les éléments (2.1, 2.2) sont pourvus, du côté de l'extrémité, de logements (3.1, 3.2) dans chacun desquels s'engagent des éléments de fixation (5) destinés à fixer, de manière amovible, le dispositif (1) à une mâchoire (6).

10. Dispositif de distraction selon la revendication 9, **caractérisé par le fait que** la séparation des éléments (2.1, 2.2) l'un de l'autre peut se faire en continu et sans paliers.

11. Dispositif de distraction selon au moins l'une des revendications 2 à 10, **caractérisé par le fait que** le dispositif (1) ou le dispositif d'actionnement (9) présente au moins un moyen d'entraînement (11) destiné à générer la pression pneumatique pour séparer éléments (2.1, 2.2) l'un de l'autre.

12. Dispositif de distraction selon au moins l'une des revendications 1 à 11, **caractérisé par le fait que** les éléments (2.1, 2.2) peuvent être déplacés l'un par rapport à l'autre par l'intermédiaire d'actionneurs piézoélectriques ou d'actionneurs en alliages à mémoire de forme.

13. Dispositif de distraction selon au moins l'une des revendications 9 à 12, **caractérisé par le fait qu'**une pluralité de logements (3.1, 3.2) sont prévus à l'élément (2.1) et/ou à l'élément (2.2) à des endroits arbitraires, éventuellement de manière déplaçable, pour la fixation et/ou le déplacement de segments d'os.
